# EUROPEAN PATENT APPLICATION

(11) **EP 4 198 447 A1**
(43) Date of publication of application: **21.06.2023**
(21) Application number: 22210234.5
(22) Date of filing: 29.11.2022
(51) Int. Cl.: G01B 9/02055, G01B 9/02091

(54) **CALIBRATION METHOD OF OPTICAL COHERENCE TOMOGRAPHY DEVICE**

(30) Priority: 20.12.2021 KR 20210182870
(71) Applicant: Huvitz Co., Ltd., Anyang-si, Gyeonggi-do 14055 (KR); Ossvis Co., Ltd., Anyang-si, Gyeonggi-do 14055 (KR)
(72) Inventor: JEONG, Hyo Sang, 14055 Anyang-Si (KR); CHO, Min Soo, 14055 Anyang-si (KR); LEE, Weon Joon, 14055 Anyang-si (KR)
(74) Representative: Henkel & Partner mbB

(57) **Abstract**

Disclosed is a calibration method of optical coherence tomography device using a non-transmissive planar target. The calibration method of an optical coherence tomography device which comprises a light source (10) that generates a measurement light (L) irradiated to the surface of a calibration target (T); a beam splitter (12) that splits the measurement light (L) into a reference light (R) and a sample measurement light (LI); a reference mirror(20) that reflects the reference light (R) and generates a reflected reference light (Rl); a scan unit (30) that reflects the sample measuring light (L1) and directs the sample measuring light (L1) to the calibration target (T); and a photodetector(40) that detects an interference light (I) formed by superimposing a reflected signal light (S) formed by reflecting the sample measurement light (L1) from the calibration target (T) and the reflected reference light (R1), and obtains the surface and inside image of the calibration target (T), comprises the steps of: obtaining three-dimensional image of the surface and the inside of the calibration target (T) by scanning the calibration target (T) using the optical coherence tomography device, and extracting a surface shape image of the calibration target (T) from the obtained the three-dimensional image; and calibrating the surface shape image of the calibration target (T) obtained by the optical coherence tomography device, according to the actual surface shape of the calibration target (T).

## Description

### CROSS-REFERENCE TO RELATED APPLICATION

This application claims benefit of priority to Korean Patent Application No. 10-2021-0182870 filed on December 20, 2021, the entire contents of which are incorporated herein by reference.

### FIELD OF THE DISCLOSURE

The present invention relates to a calibration method of optical coherence tomography device, and more particularly, to a calibration method of optical coherence tomography device using a non-transmissive planar target.

### BACKGROUND

An optical coherence tomography (OCT) device is a device that transmits measurement light to an inspection object, detects reflected light reflected from each tomographic layer on the surface and inside of the inspection object, and obtains surface and inside tomographic images of the inspection object. By using the optical coherence tomography (OCT), it is possible to obtain tomographic images of the surface and the inside of the object to be inspected with a resolution of about the wavelength of the measurement light irradiated to the object. Optical coherence tomography(OCT) is a non-invasive imaging technique and is widely used in various biomedical research and medical imaging fields, for example, medical fields such as ophthalmology, dermatology, oncology, dentistry and the like.

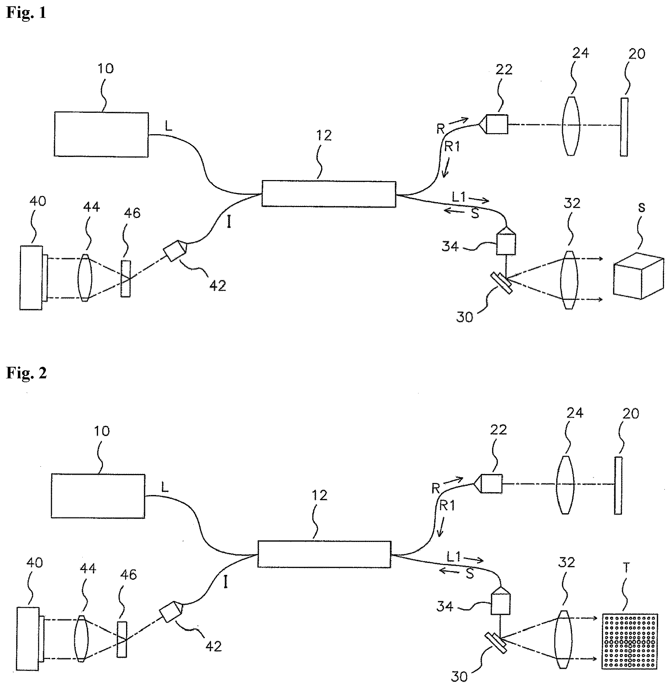

Fig. 1 is a view for illustrating the configuration of a conventional optical coherence tomography (OCT) device. As shown in Fig. 1, the conventional optical coherence tomography (OCT) device comprises a light source (10), a beam splitter (12), a reference mirror (20), a scan unit (30) and a photodetector (40). The light source (10) generates a measurement light (L). The beam splitter (12) splits the measurement light (L) into a reference light (R) and a sample measurement light (L1). The reference light (R) is irradiated to the reference mirror (20), and the sample measurement light (L1) is irradiated to the sample (s). The scan unit (30) reflects the sample measurement light (L1) and directs the sample measurement light (L1) to the inside location of the sample (s). When the sample measurement light (L1) is irradiated to the sample (s), the sample measurement light (L1) is reflected and scattered on the surface and inside layers of the sample (s), and a weak reflected signal light (S) is generated. Meanwhile, the reference light (R) is reflected by the reference mirror (20) to generate the reflected reference light (R1). The obtained reflected signal light (S) and the reflected reference light (R1) are superimposed in the beam splitter (12) to generate an interference light (I). The photodetector (40) detects the interference light (I) to obtain a tomographic image of the sample (s).

As shown in Fig. 1, in order to focus, reflect, transmit or detect various lights such as the sample measurement light (L1), the reference light (R), and the interference light (I), the conventional optical coherence tomography (OCT) device uses various optical elements such as collimators (22, 34, 42), lenses (24, 32, 44), grating (46), scan unit (30), photodetector (40) and the like. A plurality of components comprising the optical elements are assembled. In these optical elements, there is a deviation in the physical properties of the element itself, and when assembling each part, assembly deviations also occur. Therefore, for each optical coherence tomography (OCT) device, a distortion of the image obtained by measuring the sample (s) occurs. That is, when the spatial coordinates of the sample image are constructed by using the interference light (I) obtained from the sample (s), a characteristic distortion occurs due to the mechanical deviation characteristic to each device. Therefore, for each of the manufactured optical coherence tomography (OCT) device, an image of a standard sample in which the location of each part is already known is obtained with the optical coherence tomography (OCT) device, and distortions of the obtained image are corrected with the location information of the standard sample (such process is generally called "calibration") (Reference: Journal of Optics, Volume 18, Number 1, One step geometrical calibration method for optical coherence tomography, https: //iopscience.iop.org /article/10.1088/2040-8978/18/1/015301/meta).

The optical coherence tomography (OCT) device is a three-dimensional imaging device in which the information in a depth direction of the sample is obtained. Accordingly, as the standard sample used for calibration of the optical coherence tomography (OCT) device, the sample having the three-dimensional structure (steric conformation) or the sample having the three-dimensional structure through which the measurement light is transmitted is generally used. However, the sample having three-dimensional structure or the sample having three-dimensional structure through which measurement light is transmitted has disadvantages in that the calibration process is difficult, as well as sample-manufacturing is difficult, and sample-manufacturing cost is high.

### Prior Art Literature

(Patent Document 1) Korean Patent publication No. 10-2015-0056713

### SUMMARY OF THE INVENTION

### TECHNICAL OBJECTS

It is an object of the present invention to provide the calibration method of optical coherence tomography device capable of more easily correcting image distortion caused by parts themselves and assembly deviations of the parts of the optical coherence tomography device.

It is another object of the present invention to provide the calibration method of optical coherence tomography device using a target that is easy to manufacture and calibrate.

### TECHNICAL SOLUTION

In order to achieve the objects above, the present invention provides a calibration method of an optical coherence tomography device which comprises a light source (10) that generates a measurement light (L) irradiated to the surface of a calibration target (T); a beam splitter (12) that splits the measurement light (L) into a reference light (R) and a sample measurement light (LI); a reference mirror(20) that reflects the reference light (R) and generates a reflected reference light (R1); a scan unit (30) that reflects the sample measuring light (L1) and directs the sample measuring light (L1) to the calibration target (T); and a photodetector(40) that detects an interference light (I) formed by superimposing a reflected signal light (S) formed by reflecting the sample measurement light (L1) from the calibration target (T) and the reflected reference light (R1), and obtains the surface and inside image of the calibration target (T), comprising the steps of: obtaining three-dimensional image of the surface and the inside of the calibration target (T) by scanning the calibration target (T) using the optical coherence tomography device, and extracting a surface shape image of the calibration target (T) from the obtained the three-dimensional image; and calibrating the surface shape image of the calibration target (T) obtained by the optical coherence tomography device, according to the actual surface shape of the calibration target (T).

### EFFECTS OF THE INVENTION

According to the calibration method of optical coherence tomography device in accordance with the present invention, the image distortion caused by the parts themselves and assembly deviations of the optical coherence tomography (OCT) device can be corrected more simply using the target that is easy to manufacture and calibrate.

### BRIEF DESCRIPTION OF THE DRAWINGS

Fig. 1 is a view for illustrating a configuration of the conventional optical coherence tomography(OCT) device.
Fig. 2 is a view for illustrating a configuration of the optical coherence tomography device to which the calibration method in accordance with the present invention can be applied.
Fig. 3 is a view for illustrating an example of the calibration target (T) that can be used in the calibration method of optical coherence tomography device in accordance with the present invention.
Fig. 4 is a view for illustrating examples of the three-dimensional image of the calibration target (T) obtained from the optical coherence tomography device and the two-dimensional surface shape image extracted therefrom.
Fig. 5 is a view for illustrating a process of calibrating the surface shape image of the calibration target (T) obtained by the optical coherence tomography device, according to the present invention.

### DETAILED DESCRIPTION OF THE PREFERRED EMBODIMENTS

Hereinafter, the present invention will be described in detail with reference to the accompanying drawings. In the accompanying drawings, elements performing the same or similar functions as in the prior art are assigned the same reference numerals.

Fig. 2 is a view for illustrating the configuration of the optical coherence tomography device to which the calibration method in accordance with the present invention can be applied. The optical coherence tomography device, to which the calibration method of the present invention can be applied, can be a conventional optical coherence tomography device, as shown in Fig. 2, which comprises a light source (10), a beam splitter (12), a reference mirror (20), a scan unit (30), and a photodetector (40), and uses a calibration target (T) as a standard sample used for calibration.

The light source (10) generates a measurement light (L) irradiated to the surface of the calibration target (T). The measurement light (L) used for optical coherence tomography is generally broadband low-coherence light having a short coherence distance, for example, near-infrared light having a wavelength of 750 nm to 1500 nm. The beam splitter (12) splits the measurement light (L) into a reference light (R) and a sample measurement light (L1). The reference light (R) is irradiated to the reference mirror (20), and the sample measurement light (L1) is irradiated to the sample (s). The beam splitter (23) may split the measurement light (L) into, for example, the reference light (R) and the sample measurement light (L1) having an intensity of 50:50. The beam splitter (12) is also referred to as an optical coupler because it also serves to superimpose a reflected reference light (R1) and a reflected signal light (S), which will be described later. The scan unit (30) reflects the sample measurement light (L1), and induces the sample measurement light (L1) to the calibration target (T) through the objective lens (32). The scan unit (30) may use a micro electromechanical system mirror (MEMS mirror) capable of sequentially scanning the photographing locations of the target (T) by adjusting the reflection angle of the sample measurement light (L1). For example, the MEMS mirror rotates about two axes (e.g., x-axis and y-axis that are orthogonal to each other), and sequentially scans a plane (x-y plane) in which the target (T) is located. The sample measurement light (L1) is irradiated into the target (T) in a direction (z-axis direction, orthogonal to the x-axis and y-axis) perpendicular to the plane, and a three-dimensional tomography image of the target (T) can be obtained.

When the sample measurement light (L1) is irradiated to the target (T), the sample measurement light (L1) is reflected and scattered on the surface and inside of the target (T), and the reflected signal light (S) is generated. Meanwhile, the reference light (R) is reflected by the reference mirror (20) to generate the reflected reference light (R1). The generated reflected signal light (S) is induced to the scan unit (30) through the objective lens (32), and the scan unit (30) induces the reflected signal light (S) to the beam splitter (12). The reflected signal light (S) and the reflected reference light (R1) are superimposed in the beam splitter (12) to generate an interference light (I). The photodetector (40) detects the interference light (I) to obtain surface and inside image signals of the target (T). The optical coherence tomography device used in the present invention may be include optical elements such as collimators (22, 34, 42), lenses (24, 32, 44), and grating (46), if necessary, in order to focus, reflect, transmit or detect various lights such as the sample measurement light (L1), the reference light (R), and the interference light (I).

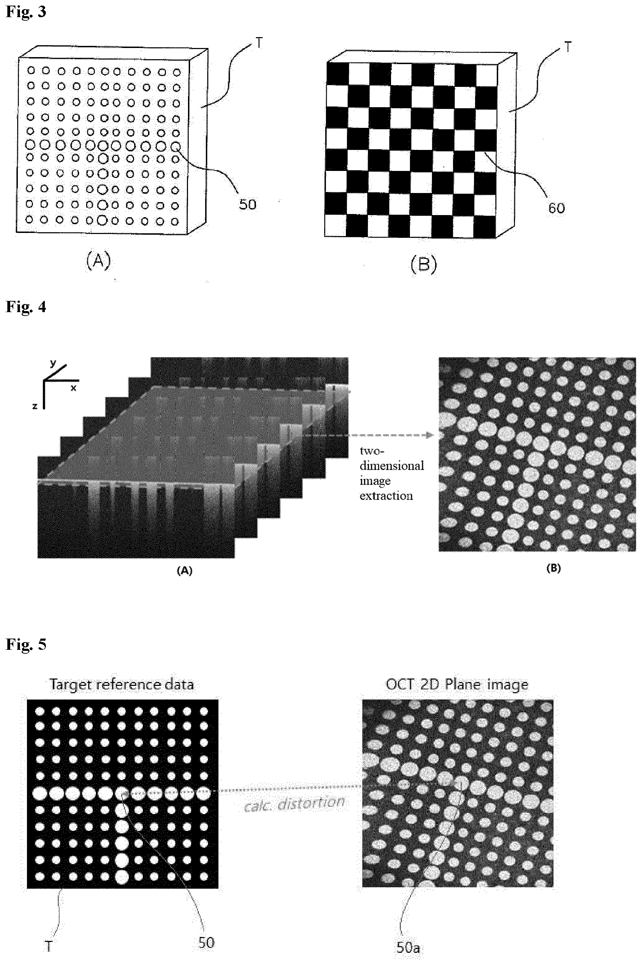

Fig. 3 is a view for illustrating an example of the calibration target (T) that can be used in the calibration method of optical coherence tomography device in accordance with the present invention. As shown in Fig. 3, the calibration target (T) that can be used in the present invention is preferably a planar target (T), and more preferably a planar target (T) in which a pattern of a predetermined shape is formed on the surface. For example, it may be a planar target (T) in which a pattern (50) in the form of a circle grid or a pattern (60) in the form of a chess board is formed on the surface. In addition, the calibration target (T) may be a non-transmissive target (T) through which the sample measurement light (L1) does not pass. Since the surface shape of the calibration target (T) or the locations of the patterns (50, 60) formed on the surface are known in advance, the calibration target (T) is used as a standard target for correcting an image obtained by the optical coherence tomography device.

Fig.4 and Fig. 5 are a view for illustrating a calibration method of optical coherence tomography device in accordance with the present invention. In order to perform calibration of the optical coherence tomography device in accordance with the present invention, first, the calibration target (T) is scanned using the optical coherence tomography device as shown in Fig. 2. Specifically, the scan unit (30) is driven along the x-y axis, a three-dimensional image of the surface and the inside of the calibration target (T) is obtained, and a surface shape image of the calibration target (T) is extracted from the obtained three-dimensional image (Surface detection). Fig. 4 is a view for illustrating an example of a three-dimensional image of the calibration target (T) obtained by the optical coherence tomography device (Fig. 4A) and a two-dimensional image of a surface shape extracted therefrom (Fig. 4B). As shown in Fig. 4, for example, when the calibration target (T) is a non-transmissive planar target in which a circle grid shape of a predetermined pattern is formed on the surface, as shown in Fig. 4A, the surface image is clear, but the inside image is a relatively opaque three-dimensional image. If the two-dimensional surface shape image is extracted therefrom, as shown in Fig. 4B, the two-dimensional image corresponding to the surface shape image of the calibration target (T) can be obtained.

Next, according to the actual surface shape of the calibration target (T), the surface shape image of the calibration target (T) obtained by the optical coherence tomography device is calibrated. As described above, the optical coherence tomography device comprises various optical elements such as lenses such as collimators (22, 34, 42), lenses (24, 32, 44), and grating (46), and image elements. Depending on the characteristics of the optical elements and the image elements, a predetermined distortion occurs in the image obtained by the optical coherence tomography device. Meanwhile, the surface shape image of the calibration target (T), specifically, the location of the pattern formed on the surface of the calibration target (T) is known in advance. Using this, the distortion of the surface shape image of the calibration target (T) obtained by the optical coherence tomography device can be corrected, that is, it can be calibrated. Fig. 5 is a view for illustrating the process of calibrating the surface shape image of the calibration target (T) obtained by the optical coherence tomography device, according to the present invention. As shown in Fig. 5, the distortion coefficient of the image obtained by the optical coherence tomography device is obtained by comparing the location of the pattern formed on the calibration target (T) in real space (namely, the real or actual calibration target (T)), for example, target reference data such as the actual location of the circle (50), with the location of the pattern in the surface shape image (namely, OCT 2D plane image) of the calibration target (T) obtained with the optical coherence tomography device, for example, the location of the circle image(50a). Using the distortion coefficient, the output image of the optical coherence tomography device is corrected, namely the output image of the optical coherence tomography device is calibrated. The calibration of the surface shape image is a process of obtaining the distortion coefficient according to the location of the image, and the distortion coefficient may be obtained according to a typical camera image calibration method (Reference: A Flexible New Technique for Camera Calibration, https://www.microsoft.com/en-us/research/wp-content/uploads/2016/02/tr98-71.pdf).

Although the present invention has been described above with reference to the accompanying drawings, the present invention is not limited to what is shown in the drawings described above. Reference numerals are labeled in the following claims to aid understanding, but the scope of the following claims is not limited to the reference numerals and what is shown in the drawings, and should be construed to encompass all modifications, equivalent constructions and functions of the exemplary embodiments.

## Claims

1. A calibration method of an optical coherence tomography device which comprises a light source (10) that generates a measurement light (L) irradiated to the surface of a calibration target (T); a beam splitter (12) that splits the measurement light (L) into a reference light (R) and a sample measurement light (L1); a reference mirror(20) that reflects the reference light (R) and generates a reflected reference light (R1); a scan unit (30) that reflects the sample measuring light (L1) and directs the sample measuring light (L1) to the calibration target (T); and a photodetector(40) that detects an interference light (I) formed by superimposing a reflected signal light (S) formed by reflecting the sample measurement light (L1) from the calibration target (T) and the reflected reference light (R1), and obtains the surface and inside image of the calibration target (T), comprising the steps of:
obtaining three-dimensional image of the surface and the inside of the calibration target (T) by scanning the calibration target (T) using the optical coherence tomography device, and extracting a surface shape image of the calibration target (T) from the obtained the three-dimensional image; and
calibrating the surface shape image of the calibration target (T) obtained by the optical coherence tomography device, according to the actual surface shape of the calibration target (T).

2. The calibration method of claim 1, wherein the calibration target (T) is a planar target (T) in which a pattern of predetermined shape is formed on the surface.

3. The calibration method of claim 2, wherein the calibration target (T) is a non-transmissive target (T) through which the sample measurement light (L1) does not pass.

4. The calibration method of claim 1, wherein the calibration of the surface shape image is carried out by comparing the location of the pattern formed on the calibration target (T) in real space with the location of the pattern in the surface shape image of the calibration target (T) obtained with the optical coherence tomography device to obtain a distortion coefficient of the image obtained by the optical coherence tomography device, and by correcting output image of the optical coherence tomography device using the distortion coefficient.
